Europäisches Patentamt

European Patent Office

Office européen des brevets

(19)

(11) **EP 1 028 705 B1**

(12) **FASCICULE DE BREVET EUROPEEN**

(45) Date de publication et mention
de la délivrance du brevet:
**05.09.2001 Bulletin 2001/36**

(21) Numéro de dépôt: **98954536.3**

(22) Date de dépôt: **06.11.1998**

(51) Int Cl.⁷: **A61K 7/48**

(86) Numéro de dépôt international:
**PCT/FR98/02382**

(87) Numéro de publication internationale:
**WO 99/24009 (20.05.1999 Gazette 1999/20)**

(54) **UTILISATIONS DU D-XYLOSE, DE SES ESTERS ET DES OLIGOSACCHARIDES CONTENANT DU XYLOSE POUR AMELIORER LA FONCTIONNALITE DES CELLULES DE L'EPIDERME**

VERWENDUNG VON D-XYLOSE, DEREN ESTERN UND VON XYLOSE ENTHALTENDEN OLIGOSACCHARIDEN ZUR VERBESSERUNG DER FUNKTIONALITÄT VON EPIDERMALZELLEN

USES OF D-XYLOSE, THE ESTERS THEREOF AND OLIGOSACCHARIDES CONTAINING XYLOSE FOR IMPROVING THE FUNCTIONALITY OF EPIDERMAL CELLS

(84) Etats contractants désignés:
**DE ES FR GB IT**

(30) Priorité: **07.11.1997 FR 9714023**

(43) Date de publication de la demande:
**23.08.2000 Bulletin 2000/34**

(73) Titulaire: **LVMH RECHERCHE**
**75008 Paris (FR)**

(72) Inventeurs:
• **DUMAS, Marc 61, Résidence le Windsor**
**F-45100 Orléans (FR)**

• **BONTE, Frédéric**
**F-45100 Orléans (FR)**

(74) Mandataire: **Giraud, Françoise et al**
**Cabinet Beau de Loménie**
**158, rue de l'Université**
**75340 Paris Cedex 07 (FR)**

(56) Documents cités:
**CH-A- 368 267          CH-A- 642 851**

• **DATABASE WPI Section Ch, Week 9633 Derwent Publications Ltd., London, GB; Class D17, AN 96-329415 XP002071196 & JP 08 151313 A (KANEBO LTD)**

**Description**

L'invention concerne de nouvelles utilisations du D-xylose, de ses esters et des oligosaccharides contenant du xylose pour améliorer la fonctionnalité des cellules de l'épiderme.

Elle concerne plus précisément de nouvelles utilisations de ces produits dans le domaine de la cosmétique et de la pharmacie, notamment de la dermatologie, visant à améliorer cette fonctionnalité, par stimulation de la synthèse des protéoglycannes et/ou des glycosaminoglycannes.

Dans l'ensemble du présent mémoiré, on désignera les protéoglycannes par l'abréviation PG et les glycosaminoglycannes par l'abréviation GAG.

Lorsque l'on souhaite améliorer l'hydratation de la peau, on peut faire appel à des produits dont l'action est reliée :

- soit à un effet occlusif : cette propriété permet de réguler la perte en eau par création d'une barrière qui s'oppose au passage des molécules d'eau,
- soit à un effet humectant : ces produits agissent par hygroscopie,
- soit à un effet de différenciation des kératinocytes conduisant à une hydratation de la peau : cette action permet d'aboutir à la formation des coméocytes, en particulier de l'enveloppe cornée et à celle des substances synthétisées par les kératinocytes au cours de leur différenciation,
- soit à un effet d'augmentation du pouvoir de rétention d'eau des espaces intercellulaires ; cet effet peut être obtenu en favorisant la synthèse des PG et/ou des GAG par les cellules de la peau, notamment par les kératinocytes et/ou les fibroblastes.

Les PG, autrefois appelés mucopolysaccharides sont des macromolécules complexes constituées d'une protéine centrale, également appelée « protéine porteuse », substituée au niveau de ses résidus sérine avec des chaînes osidiques appelées GAG. Ces chaînes consistent en une succession d'hcxosamines (glucosamine ou galactosamine) en alternance avec un autre sucre (glucuronique ou iduronique). Dans la peau, elles sont sulfatées en position 2, 4, 6 ou sur la fonction aminc. (*Structure and metabolism of proteoglycans and glycosaminoglycans, J. E. Silbert, J. Invest. Dermatol, (1982), 79, 31-37).*

Les PG. tout comme les GAG, sont sécrétés dans la peau par les kératinocytes et les fibroblastes et sont responsables pour partie de son hydratation.

L'organisme est soumis en permanence à de nombreuses agressions de la part du milieu extérieur.

La peau remplit un rôle de protection de l'organisme contre le milieu extérieur. L'épiderme est la barrière extérieure de la peau. Il est constitué notamment de kératinocytes, qui se différencient au cours de leur migration de la couche basale de l'épiderme jusqu'à la couche cornée de l'épiderme.

Une des fonctions les plus importantes de cette barrière qu'est l'épiderme, est d'empêcher la perte d'eau par l'organisme. C'est également de combattre les diverses agressions physiques, chimiques et microbiennes du milieu environnant.

La lutte contre la perte d'eau est assurée en grande partie par les PG et les GAG, présents en grande quantité dans le milieu extra-cellulaire. Ces molécules participent de manière importante à l'hydratation et à la minéralisation (fixation de contre-ions) des matrices extra-cellulaires grâce notamment à leur capacité de fixer de l'eau. Elles participent également activement au bon fonctionnement des cellules de l'épiderme.

Ces PG et GAG sont, en particulier, synthétisés par les kératinocytes dans l'épiderme.

Ces PG et GAG, une fois synthétisés par une cellule, restent dans la matrice extra-cellulaire entourant cette cellule et ne migrent pas ou peu.

Les PG représentent environ 0,2 % du poids sec de la peau, ce qui est peu en comparaison de la quantité de collagène par exemple qui représente plus de 75% de ce poids sec (*The collagens : biochemistry and physiopathology, E.J. Kucharz, Ed. Springer-Verlag, 1992*). Cela ne reflète en rien l'importance physiologique des PG et des GAG.

En effet, par leurs structures osidiques polyanioniques, ces molécules ont la particularité de s'associer très fortement avec des contre-ions (sodium et potassium) et des molécules d'eau. Elles ont la capacité de fixer de l'eau jusqu'à 1000 fois leur volume, formant ainsi des gels. Elles se comportent donc comme des hydratants naturels et minéralisants de l'épiderme, en particulier des cellules de l'épiderme, notamment des cellules germinatives, des membranes basales cellulaires, des annexes cutanées en particulier des follicules pileux et également des matrices extra-cellulaires comme celle de la jonction dermo-épidermique (JDE). Les PG et les GAG jouent donc un rôle déterminant dans la fonctionnalité de ces cellules et de la matrice sur laquelle ces cellules adhèrent.

Les GAG les plus abondants de la peau sont l'acide hyaluronique (50% de l'acide hyaluronique de l'organisme est dans là peau) et le dermatan sulfate, alors que d'autres sont retrouvés en plus petite quantité comme les chondroitin-4 et -6-sulfarc, l'heparan sulfate et le keratan sulfate. L'acide hyaluronique est sans doute l'agent hydratant de la peau lé plus connu. On notera, qu'à la différence des autres GAG, cette molécule n'est pas sulfatée et n'est pas non plus fixée à une protéine porteuse.

L'épiderme contient de la chondroitin-4-sulfate et du keratan sulfate. La jonction dermo-épidermique contient de la chondroitin-6-sulfate et de l'heparan sulfate, qui sont très importants dans la cohésion entre le derme et l'epiderme. L'acide hyaluronique est localisé principalement dans la couche basale ct de façon moindre dans la couche spineuse *(Age-dependent changes of hyaluronan in human skin*, *L.J.M. Meyer and R. Stern, J. Invest. Dermatol., (1994), 102, 385-389*).

On sait aussi que le vieillissement a un retentissement important sur la teneur et la distribution des PG et des GAG dans la peau. L'acide hyaluronique, voit sa distribution épidermique chuter de manière drastique au point de n'être plus détecté dans l'épiderme sénescent. (*Age-dependent changes of hyaluronan in human skin. L.J.M. Meyer and R. Stern. J. Invest. Dermatol., (1994), 102, 385-389*). De la même manière on note une diminution de: la chondroitin-6-sulfate localisée principalement dans la Jonction Dermo-Epidermique (JDE) (*Patterns of glycosaminoglycan/proteoglycan immunostaining in human skin during ageing*, *M.D. Willen, J. Invest. Dermatol., (1996), 968-974*).

Le D-xylose est un sucre bien connu de la: famille des aldoses à 5 carbones. Pour sa description, ses sources et ses principales propriétés, on pourra se reporter à la publication du Merck Index 12ème édition (1996), n° 10220.

I.e brevet suisse CH 368 267 décrit des compositions à effet hydratant contenant, en combinaison, un pentose et un acide aminé. Le xylose est cité parmi les pentoses en tant que produit à caractère hygroscopique.

Dans le cadre de la présente invention, on a maintenant découvert de manière surprenante que le D-xylose, ses esters ainsi que les oligosaccharides contenant du xylose permettaient de stimuler de manière importante et significative la synthèse et la sécrétion des PG et des GAG par les kératinocytes humains.

Ainsi, le D-xylose, ses esters et les oligosaccharides contenant du xylose, grâce à leur action stimulante de la synthèse des PG et des GAG par les kératinocytes épidermiquss peuvent être utilisés dans des compositions à usage topique cosmétiques ou pharmacentiques, notamment dermatologiques, permettant, entre autres, de favoriser l'hydratation de la peau, en particulier celle de l'épiderme, mais aussi des tissus possédant un compartiment kératinocytaire de revêtement comme les muqueuses en particulier des lèvres, et également les annexes cutanées en particulier les follicules pileux.

Le D-xylose, ses esters et les oligosaccharides contenant du xylose peuvent être aussi utilisés pour prévenir ou traiter les effets du vieillissement cutané, en particulier les effets sur l'épiderme qui est le siège d'un fort déclin de la teneur en PG et en GAG avec l'âge, s'accompagnant eu particulier d'une plus grande sécheresse cutanée.

Le D-xylose ainsi que ses esters et les oligosaccharides contenant du xylose peuvent ainsi être utilisés dans toutes les applications où l'on cherche à améliorer la synthèse des GAG et des PG.

Ainsi, selon l'une de ses caractéristiques, l'invention concerne l'utilisation d'un composé choisi dans le groupe constitué du D-xylose, de ses esters et des oligosaccharides contenant du D-xylose, comme agent cosmétique destiné à stimuler la synthèse et/ou la sécrétion des protéoglycannes (PG) et/ou des glycosaminoglycannes (GAG) par les kératinocytes, notamment les kératinocytes de l'épidcrme et des tissus possédant un compartiment kératinocytaire de revêtement tels que les muqueuses, en particulier les lèvres, et les annexes cutanées, en particulier les follicules pileux, ledit agent étant incorporé dans une composition cosmétique comprenant un véhicule cosmétiquement acceptable.

Plus précisément, il est apparu que le D-xylose ainsi que les composés cités précédemment, du fait de leur effet de stimulation de la synthèse des GAG et des PG par les kératinocytes humains normaux permettaient :

- de lutter contre le vieillissement de l'épiderme. Il est en effet connu que le vieillissement de l'épiderme est, pour une part importante, lié à une perte d'acide hyaluronique,
- de lutter contre le dessèchement de la peau lié à une insuffisance de l'action des GAG, en particulier de l'acide hyaluronique. Un tel dessèchement est en particulier observé sur les peaux âgées et est lié essentiellement à une perte d'acide hyaluronique,
- d'améliorer la tonicité de la peau. Il a été en effet observé que l'augmentation de la synthèse des GAG permettait de créer un environnement cellulaire hydraté favorable aux échanges de nutriments, d'ions, de cytokine et de facteurs de croissance sécrétés par des cellules épidermiques. Un tel environnement est favorable aussi à l'élimination des métabolites toxiques. Cet effet se traduit donc par une peau tonique et en bonne santé,
- de maintenir ou restaurer la souplesse et l'élasticité de la peau. Cet effet est lié à la stimulation de la synthèse des GAG qui permet de créer un environnement hydraté pour les constituants matriciels, en particulier au niveau de la jonction dermo-épidermique pour favoriser les micro-déplacements entre les éléments de cette matrice lors d'un stress mécanique. Un tel effet contribue donc à faire une peau plus souple et plus élastique,
- d'améliorer la minéralisation de l'épiderme, rendant ainsi la peau plus saine et améliorant sa vitalité. Cet effet est lié à l'amélioration de la synthèse des GAG qui permet d'assurer une bonne minéralisation de l'épiderme. En effet les GAG, par leurs groupements chargés, peuvent fixer les ions et contribuer à l'osmolarité de l'épidemie. Là encore, une bonne minéralisation de la peau est synonyme de peau saine préscntant une bonne vitalité,

- de faciliter les échanges intercellulaires. Cet effet est à rapprocher également de la stimulation de la synthèse des GAG qui permet d'assurer une différenciation correcte de l'épidenne puisqu'une destruction de l'acide hyaluronique provoque une ouverture des espaces intercellulaires et une acanthose épidermique. Cet effet permet d'obtenir une peau plus tonique, plus dense et plus compacte,
- d'améliorer la structure tridimensionnelle de la jonction dermoépidermique. Ceci est également à relier à l'amélioration de la synthèse des GAG qui permet d'assurer l'organisation spatiale des constituants matriciels en assurant, par exemple, au niveau de la jonction dermo-épidermique, le renforcement de la liaison entre la laminine-6 et le nidogène,
- de faciliter la cicatrisation sans formation de cicatrices, permettant ainsi la réparation de mioro-traumatismes épidcrmiques qui apparaissent lorsqu'il y a rupture de la continuité cutanée. Un tel effet permet de lutter contre les gerçures et l'aspect craquelé de la peau,
- de faciliter la migration des kératinocytes, permettant la formation d'une couche cornée de bonne qualité,
- de moduler l'action des facteurs de croissance et des cytokines produits par les cellules de la peau. Un tel effet permet aux cellules de disposer des signaux dont elles ont besoin pour assurer leur fonction.

Selon l'une de ses autres caractéristiques, l'invention concerne également des utilisations du D-xylose et/ou d'un de ses esters pour la fabrication d'une composition pharmaceutique, notamment dermatologique, destinée à traiter les insuffisances de la synthèse ou de la sécrétion des protéoglycannes (PG) et/ou des glycosaminoglycannes (GAG) par les kératinocytes, en vue de corriger les effets négatifs desdites insuflisances, en particulier d'améliorer l'état fonctionnel des cellules de la peau, notamment de l'épiderme.

Cette composition pharmaceutique pourra, en particulier, être destinée à faciliter la cicatrisation et à réparer les mioro-traumatismes épidermiques ou à traiter les ulcérations cutanées, en particulier les ulcérations des jambes ou à réduire les striae distensae de la grossesse.

Selon une variante particulièrement avantageuse, on choisira le D-xylose qui présente l'avantage d'être un produit commercial.

Toutefois, on pourra également recourir à l'un de ses esters, en particulier l'ester de l'acide D-galacturonique.

On pourra également utiliser avantageusement des esters d'acides gras comprenant de 16 à 24 atomes de carbone, en particulier les esters d'acides gras naturels.

Parmi ces esters d'acide gras, on choisira avantageusement les esters des acides palmitique, stéarique, oléique, linoléique, linolénique, arachidonique, érucique, lignocérique.

Ces esters d'acide gras du D-xylose présentent un intérêt tout particulier pour l'invention car, du fait de la présence de la chaîne grasse, ils permettent une meilleure pénétration du xylose dans la peau en rendant l'ensemble du composé lipophile. Par ailleurs, du fait de la présence des estérases dans la peau, le xylose peut se libérer dans la peau, permettant ainsi à ces produits d'avoir les mêmes avantages que le D-xylose sur la synthèse et la sécrétion des GAG.

Une autre famille de composés dont l'utilisation s'avère particulièrement intéressante pour l'invention est constituée de la famille des oligosaccharides contenant du xylose. Par oligosaccharide au sens de l'invention, on entend des chaînes de sucres contenant de 2 à 6 sucres.

On choisira de préférence, selon la présente invention, l'oligosaccharide contenant du xylose parmi les oligosaccharides tels que définis précédemment comprenant au moins un xylose et comprenant de 1 à 6 sucres. De tels oligosaccharides sont avantageusement choisis parmi le xylobiose, le xylobiose hexaacétate, le méthyl-β-xylobioside, le xylotriose, le xylotétraose, le xylopentaose et le xylohexaose.

Les oligosaccharides seront de préférence le xylobiose qui est composé de deux molécules de xylose liées par une liaison 1-4 ainsi que les acétates de xylobiose tels que le xylobiose hexaacétate.

Ces oligosaccharides sont particulièrement intéressants dans le cadre de l'invention car ils peuvent être dégradés progressivement dans la peau par les nombreuses osidases qui s'y trouvent et libérer ainsi du xylose. Ainsi donc, ces oligosaccharides contenant du xylose agissent, en quelque sorte, comme des précurseurs de xylose et constituent de véritables réservoirs de xylose dont la libération se trouvera étalée dans le temps.

Dans les compositions aussi bien cosmétiques que pharmaceutiques de l'invention, le D-xylose ou ses esters ou précurseurs de type oligosaccharide défini précédemment seront avantageusement présents à une concentration comprise entre 0,001 et 5 %, de préférence entre 0,01 et 0,5 % en poids par rapport au poids de ladite composition.

D'une façon générale, les compositions de l'invention seront avantageusement formulées pour une application topique. Il pourra en particulier s'agir d'émulsions, de lotions, de gels, de rouges à lèvres, de mascaras.

Selon une autre variante de l'invention, la composition cosmétique ou pharmaceutique, notamment dermatologique, pourra comprendre des liposomes, le D-xylose, l'ester de D-xylose ou l'oligosaccharide contenant du xylose, se trouvant soit à l'extérieur desdits liposomes, soit partiellement ou totalement incorporé dans lesdits liposomes.

Enfin, selon une variante particulièrement intéressante de l'invention, le D-xylose ou ses esters ou précurseurs de type oligosaccharide défini précédemment pourront être combinés dans la composition de l'invention avec un certain nombre de principes actifs tels que :

- des sucres aminés, en particulier la D-glucosamine et la D- galactosamine ;
- des acides aminés, en particulier la L- sérine ou tout extrait en contenant, en particulier des extraits d'algues ;
- de l'ascorbate de sodium (vitamine C) et de ses dérivés comme le phosphate, le palmitate, l'acétate ou le propionate ;
- de la vitamine A (appelé aussi rétinol), de ses esters et dérivés en particulier le palmitate, l'acétate et le propionate ;
- de l'acide madécassique, de l'acide asiatique et de leurs dérivés glycosylés;
- des vitamines du groupe B, en particulier les vitamines B1, B6 et B12 et l'acide folique ;
- de la vitamine PP ;
- de la forskoline, de ses dérivés et des extraits de coléus forskolii ;
- de l'acide salicylique ct ses dérivés, en particulier ceux à chaînes lipophilcs;
- l'ecdystérone et ses dérivés, en particulier les acétates ;
- des rétinoïdes, en particulier l'acide rétinoïque, le rétinaldéhyde et le rétinylphosphate ;
- des oligo-éléments, tels que la silice et ses dérivés comme le silanol, le magnésium et ses sels, le manganèse et ses sels ;
- des extraits de Potentilla erecta ;
- des extraits de fruits, en particulier de pomme et de citron, en particulier l'eau de pomme ou ses polyphénols ;
- des inhibiteurs des phosphodiestérases, en particulier les xanthines;
- des extraits de Siegesbeckia orientalis ou de Daturoside ;
- des extraits de Centella asiatica enrichi en triterpènes ;
- des extraits de Bertholletia ;
- de l'acide chlorogénique, de l'acide caféique, de l'acide sinapique et de l'acide cafféoyl quinique ;
- des substances antiradicalaires, cn particulier les oligomères procyanidoliques, les extraits de thé vert, de pépins de raisins, l'acide nordihydroguaiarétique (NDGA), les extraits de cureumine et les curcuminoides.

Selon une autre de ses caractéristiques : essentielles, l'invention conceme également un procédé de traitement cosmétique ou pharmaceutique selon lequel on cherche à stimuler la synthèse et/ou la sécrétion des protéoglycannes (PG) et/ou des glycosaminoglycannes (GAG). Selon ce procédé, on applique sur la partie du corps concerné une quantité efficace de D-xylose ou d'un de ses esters ou d'un oligosaccharide contenant du D-xylose tels que définis précédemment, pour obtenir la stimulation desdites synthèse et sécrétion.

Les compositions utilisées dans ces procédés de traitement sont celles décrites précédemment.

Selon une autre caractéristique essentielle de l'invention, ellc concerne également l'utilisation d'un ester du D-xylose, en particulier de l'ester de l'acide D-galacturonique, ou d'un des esters d'acide gras du D-xylose tel que défini précédemment ou d'un oligosaccharide contenant du D-xylose, en particulier d'un oligosaccharide tel que défini précédemment, comme agent cosmétique destiné à hydrater la couche basale de l'épiderme.

Elle concerne également selon un autre aspect, l'utilisation d'un ester du D-xylose, en particulier de l'ester de l'acide D-galacturonique, ou d'un des esters d'acide gras du D-xylose tel que défini précédemment, ou d'un oligosaccharide contenant du D-xylose, en particulier d'un oligosaccharide tel que défini précédemment, pour la fabrication d'une composition pharmaceutique, notamment dermatologique, destinée à traiter les insuffisances de l'hydratation de la couche basale de l'épiderme.

Les exemples suivants sont donnés à titre purement illustratif de l'invention.

EXEMPLES

Exemple 1

1.1- Principe général du test.

Des cultures de kératinocytes humains normaux ont été réalisées in-vitro, en présence de D-xylose, dans un milieu contenant deux traceurs radioactifs qui s'incorporent dans le, chaînes osidiques des PG et des GAG lors de la synthèse de ces molécules.

Ces traceurs permettent de mesurer la quantité de PG et de GAG.

Il existe deux familles de GAG qui diffèrent par leurs chaînes sucrées. L'une est à base d'enchaînement de glucosamines et l'autre à base d'enchaînement de galactosamines. C'est pourquoi on a utilisé deux traceurs radioactifs différents, la $^3$H-D-glucosamine et la $^{14}$C-D-galactosamine, qui s'incorporent dans les chaînes osidiques des PG et des GAG au cours de leur synthèse. Ce protocole permet de bien évaluer la synthèse de l'ensemble des GAG et, accessoirement, de montrer que le xylose agit de manière globale sur l'ensemble des synthèses de ces familles moléculaires.

1.2- Protocole détaillé de mise en oeuvre du test.

Ensemencement des cellules:

Pour mettre en évidence l'effet du D-xylose, on ensemence 25000 kératinocytes humains normaux dans des puits de culture (plaque de 96 puits Falcon. 25000 kératinocytes par puits) dans 100 μl de milieu de culture pour kératinocytes (K-SFM, Gibco). Les cellules sont incubées 24 h à 37°C dans une atmosphère saturée en humidité avec 5% de $CO_2$.

Traitement des cellules:

Au bout de ces 24 heures, le milieu d'ensemencement est remplacé par 100 μl de milieu K-SFM avec addition de D-xylose à des concentrations non cytotoxiques de 1, 5 et 10 mM (solution aqueuse de la molécule introduite à 0,1% v/v) avec deux traceurs radioactifs, à savoir la $^3$H-D-glucosamine et la $^{14}$C-D-galactosamine (4μCi/ml, Amersham). Dans les cultures témoin, la non-utilisation de D-xylose est compensée par un ajout à 0,1% v/v d'eau. Les cultures sont alors incubées 48h à 37°C en atmosphère saturée en humidité et avec 5% de $CO_2$.

Dosage des PG et des GAG sécrétés.

Les surnageants de culture sont collectés, réunis deux à deux pour avoir un volume d'échantillon plus important, et traités avec 200 μl de pronase à 0.2 mg/ml (Sigma) pendant 17h à 37°C afin d'hydrolyser la protéine porteuse et libérer les GAG des PG. L'enzyme est ensuite inactivée par chauffage pendant 10 min dans de l'eau bouillante. Puis on revient à température ambiante. Une précipitation sélective des GAG est réalisée avec 40 μl de chlorure de cétyl pyridinium (CPC à 100 m/ml, Sigma) en présence de 40 μl d'un mélange d'acide hyaluronique (Fluka) de dermatan sulfate (Fluka) et de chondroitin sulfate (Sigma) tous à 2 mg/ml (ce qui fait un ajout d'une solution contenant 6 mg/ml de GAG non radioactifs). On ajoute ces GAG non radioactifs pour déclencher la précipitation et entraîner les GAG radioactifs (qui pris seuls seraient en quantité insuffisante pour précipiter) dans le précipité. Le précipité obtenu est lavé 2 fois avec 400 μl d'une solution de CPC à 10 mg/ml, puis solubilisé dans 500 μl de méthanol (Merck). La radioactivité est finalement mesurée en scintillation liquide avec 10 ml de liquide scintillant (Packard).
Il faut noter que dans l'exploitation des résultats, les GAG dont on a mesuré la synthèse ont deux origines. Ils proviennent à la fois des GAG synthétisés et sécrétés et des PG synthétisés et sécrétés (qui ont subi une hydrolyse par la pronase pour les besoins du dosage).

Dosage des protéines cellulaires:

Afin de ramener la radioactivité à une quantité unitaire de matériel cellulaire, un dosage des protéines cellulaires est effectué. Après élimination du milieu de culture et deux rinçages avec un tampon phosphate pH=7,2 (PBS, Gibco), le tapis cellulaire des puits de culture est dissous dans 30 μl de NaOH 0,1M à 37°C et les plaques agitées pendant 30 mn à 37°C. Dans chaque puits on ajoute 200 μl de réactif à l'acide biccinchoninique/sulfate de $Cu^{++}$ (Kit Sigma). On fait incuber pendant 30 min à 37°C et à l'obscurité. Les protéines que l'on veut doser réduisent les ions cuivrique ($Cu^{++}$) du sulfate de cuivre en ions cuivreux ($Cu^+$). Ces derniers forment avec l'acide biccinchoninique des complexes chromogènes absorbant à 570 nm, qui est la longueur d'onde utilisée pour la mesure de la densité optique. Parallèlement, une gamme d'étalonnage est réalisée dans les mêmes conditions expérimentales avec de l'albumine sénque bovine (BSA, Sigma) de 0 à 100 μg/puits, afin de traduire les densités optiques (DO) obtenues avec les protéines cellulaires en μg d'équivalent BSA par puits.

Expression des résultats:

Les quantités de GAG mesurées seront ramenées à une quantité unitaire de protéine (exprimée en μg d'équivalent BSA).
L'activité « A » de stimulation de la sécrétion des GAG est calculée sous forme d'un pourcentage, selon la formule suivante :

$$A = \left( \frac{q_p - q_t}{q_t} \right) \times 100$$

Dans laquelle $q_p$ et $q_t$, respectivement pour les cultures traitées par le D-xylose et pour les cultures témoin non traitées, représentent la quantité de GAG libérée, exprimée en nombre de désintégrations par minute (dpm) et ramenée à 1μg de protéine équivalent BSA.

Statistiques:

Le test t de student sera effectué pour déterminer si les sécrétions de PG et de GAG en présence de D-xylose sont significativement stimulées comparées aux cultures témoins, et pour comparer les valeurs de viabilité cellulaire obtenue entre cellules témoins et traitées.

1.3- Produit testé.

D-xylose pur à 99% (SIGMA ; référence : X3877)

1.4- Résultats du test.

**Stimulation de synthèse et de la sécrétion des GAG et des PG par le D-xylose.**

En utilisant la $^3$H-D-glucosamine comme traceur de la synthèse des PG et des GAG.

| D-xylose cn mM | GAG dpm/μg de protéine | A(%) | test t de Student valeur de p |
|---|---|---|---|
| 0 | 1387±116 | | |
| 1 | 2148±160 | 55 | <0,0001 (S) |
| 5 | 2661±202 | 92 | <0,0001 (S) |
| 10 | 2950±262 | 113 | <0,0001(S) |

En utilisant la $^{14}$C-D-galactosamine comme traceur de la synthèse des PG et des GAG.

| D-xylose en mM | GAG dpm/ug dû protéine | A(%) | test t de Student valeur de p |
|---|---|---|---|
| 0 | 102±8 | | |
| 1 | 136±12 | 34 | <0,0001 (S) |
| 5 | 209±15 | 105 | <0,0001 (S) |
| 10 | 250±28 | 145 | <0,0001 (S) |

S: différences significatives entre cultures témoins et traitées (p<0,05)
NS: différences non significatives entre témoins et traités (p>0,05)

1-5-Analyse et discussion des résultats.

Il apparaît, à la lecture des tableaux, une augmentation importante et significative des GAG radioactifs sécrétés dans les cultures traitécs par le D-xylose par comparaison avcc les cultures témoins.
On voit que le D-xylose stimule très fortement la synthèse, ainsi que la sécrétion extracellulaire, des PG et des GAG par les kératinocytes de l'épiderme humain et ce, quel que soit le traceur radioactif utilisé pour suivre cette synthèse.
Compte tenu des propriétés déjà décrites des PG et des GAG, le D-xylose apparaît comme un agent particulièrement intéressant en cosmétique et en pharmacie en stimulant la synthèse et la sécrétion des PG et des GAG, et permettant de ce fait d'obtenir une restauration ou une régulation de l'hydratation de l'épiderme et une meilleure fonctionnalité des cellules épidermiques.

**Exemple 2 :** Emulsion hydratante anti-âge.

| D-xylose | 0,2g |
|---|---|
| Céramides de blé | 0,2g |
| Protéines de blé | 1g |
| Extrait de pomme | 1g |
| Palmitate de vitamine A | 0,01g |
| Acétate de vitamine E | 0,1g |
| Excipient pénétrant avec conservateurs et parfums | qsp 100g |

Cette émulsion est utilisée en application topique quotidienne sur le visage. Cette préparation nourrissante améliore l'hydratation cutanée, la finesse de la peau, le grain de la peau et atténue les rides.

**Exemple 3 :** Gel hydratant raffermissant.

| D-xylose | 0,3g |
|---|---|
| Extrait d'algue rouge | 0,5g |
| Extrait de Centella asiatica | 0,1g |
| Extrait de Bertholletia | 0,05g |
| Vitamine C magnésium phosphate | 0,1g |
| Excipient avec conservateurs et parfums | qsp 100g |

Ce gel hydratant est utilisé en application topique quotidienne sur le visage, le cou et le buste. Il exerce un effet tenseur sur la peau et en améliore la souplesse.

**Exemple 4 :** Gel liposomal hydratant et réparateur.

| D-xylose | 0,2g |
|---|---|
| Lécithine de soja | 2g |
| Protéines de blé | 3g |
| Acétate de vitamine A | 0.01g |
| $\alpha$-tocophérol | 0,01g |
| Excipient avec conservateurs et parfums | qsp 100g |

Ce gel liposomal est utilisé en application qubtidienne, de préférence le soir et sur le visage. Cette composition améliore la souplesse de la peau et son hydratation pour sa régénération.

**Exemple 5 :** Lotion hydratante er tonifiante.

| D-xylose | 0,2g |
|---|---|
| Extrait de Panax ginseng | 0,2g |
| AMP cyclique | 0,05g |
| Théophylline | 0,1g |

(suite)

| Excipient avec conservateurs et parfums | qsp 100g |
|---|---|

Cette lotion hydratante est utilisée en application topique quotidienne sur le visage de préférence le marin pour une peau plus belle, plus éclatante.

**Exemple 6 :** Mascara hydratant.

| D-xylose | 0,3g |
|---|---|
| Acide hyaluronique | 0,5g |
| Pigments colorés | 10g |
| Cires | 30g |
| Excipient | qsp 100g |

Ce maquillage traitant pour le visage permet d'hydrater la peau et de l'assouplir tout en masquant les ridulcs superficielles pour un aspect plus soyeux..

**Exemple 7 :** Gel liposomal hydratant et réparateur.

| D-xylose | 0,5g |
|---|---|
| Phosphate de vitamine E | 1g |
| Oxydes de fer | 8g |
| Billes de nylon Orgasol | 3g |
| Excipient émulsion | qsp 100g |

Ce maquillage de soin pour le visage en application quotidienne permet d'assouplir la peau et de l'hydrater tout en la protégeant.

**Exemple 8 :** Fluide protecteur

xylose : 0,5
céramide 3 : 0,02
acétate de DL alpha tocophérol : 0,2
méthoxycinnamate d'octyle : 7,5
uvinul M40® : 2,5
excipient gel fluide avec conservateur qsp 100 g

Ce produit présente une nette activité hydratante et raffermissante de la peau, liée à l'action d'augmentation de la synthèse des GAG.

**Exemple 9 :** émulsion crème douce

xylose : 0,5
céramide 3 : 0,02
peptides de lupin : 3
Oligomeres procyanidoliques (OPC) : 0,5
excipient crème avec conservateurs et parfums qsp 100 g

Ce produit présente une nette activité hydratante et raffermissante de la peau, liée à l'action d'augmentation de la synthèse des GAG.

**Revendications**

1.   Utilisation cosmétique d'un composé choisi dans le groupe constitué du D-xylose, de ses esters et des oligosaccharides contenant du D-xylosc, comme agent cosmétique stimulant la synthèse et/ou la sécrétion des protéoglycannes (PG) et/ou des glycosaminoglycannes (GAG) par les kératinocytes, notamment les kératinocytes de l'épiderme et des tissus possédant un compartiment kératinocytaire de revêtement tels que les muqueuses, en particulier les lèvres, et les annexes cutanées, en particulier les follicules pileux, ledit agent étant incorporé dans une composition cosmétique comprenant un véhicule cosmétiquement acceptable.

2.   Utilisation selon la revendication 1, **caractérisée en ce que** ledit agent cosmétique contribue à au moins une des activités suivantes :

   -   lutter contre le vieillissement de l'épiderme,
   -   lutter contre le dessèchement de la peau lié à une insuffisance de l'action des GAG, en particulier de l'acide hyaluronique,
   -   améliorer la tonicité de la peau,
   -   maintenir ou restaurer la souplesse et l'élasticité de la peau,
   -   améliorer la minéralisation de l'épiderme, rendant ainsi la peau plus saine et améliorant sa vitalité,
   -   faciliter les échanges intercellulaires,
   -   améliorer la structure tridimensionnelle de la jonction dermoépidermique,
   -   faciliter la cicatrisation sans formation de cicatrices, permettant ainsi la réparation de micro-traumatismes épidermiques,
   -   faciliter la migration des kératinocytes, permettant la formation d'une couche cornée de bonne qualité,
   -   moduler l'action des facteurs de croissance et des cytokines produits par les cellules de la peau.

3.   Utilisation du D-xylose et/ou d'un de ses esters pour la fabrication d'une composition pharmaceutique, notamment dermatologique, destinée à traiter les insuffisances de la synthèsc ou de la sécrétion des protéoglycannes (PG) et/ou des glycosaminoglycannes (GAG) par les kératinocytes, en vue de corriger les effets négatifs desdites insuffisances, en particulier d'améliorer l'état fonctionnel des cellules de la peau, notamment de l'épiderme.

4.   Utilisation selon la revendication 3, **caractérisée en ce que** ladite composition pharmaceutique, notamment dermatologique est destinée à faciliter la cicatrisation et à réparer les micro-traumatismes épidermiques ou à traiter les ulcérations cutanées, en particulier des ulcérations des jambes, ou à réduire les striae distensae de la grossesse.

5.   Utilisation selon l'une des revendications 1 à 4, **caractérisée en ce que** ledit composé est le D-xylose.

6.   Utilisation selon l'une des revendications 1 à 4, **caractérisée en ce que** ledit composé est un ester de l'acide D-galacturonique.

7.   Utilisation selon l'une des revendications 1 à 4, **caractérisée en ce que** ledit composé est un ester d'acide gras comprenant de 16 à 24 atomes de carbone, en particulier un ester d'un acide gras naturel.

8.   Utilisation selon la revendication 7, **caractérisée en ce que** ledit acide gras est choisi dans le groupe constitué des acides palmitique, stéarique, oléique, linoléique, linolénique, arachidonique, érucique, lignocérique.

9.   Utilisation selon l'une des revendications 1 à 4, **caractérisée en ce que** ledit composé est un oligosaccharide comprenant au moins un xylose et comprenant de 1 à 6 sucres, choisi dans le groupe constitué du xylobiose, du xylobiose hexaacétate, du methyl-β-xylobioside, du xylotriose, du xylotétraose, du xylopentaose et du xylohexaose.

10.   Utilisation selon l'une des revendications 1 à 9, **caractérisée en ce que** ledit composé est présent dans ladite composition à une concentration comprise entre 0,001 et 5 %, de préférence entre 0,01 et 0,5 %, en poids par rapport au poids de ladite composition.

**11.** Utilisation selon l'une des revendications 1 à 10, **caractérisée en ce que** ladite composition est formulée pour une application topique.

**12.** Utilisation selon l'une des revendications 1 à 11, **caractérisée en ce que** ladite composition contient des liposomes, ledit composé se trouvant soit à l'extérieur desdits liposomes, soit partiellement ou totalement incorporé dans lesdits liposomes.

**13.** Utilisation selon l'une des revendications 1 à 12, **caractérisée en ce que** ladite composition contient en outre au moins un autre principe actif choisi dans le groupe constitué :

- des sucres aminés, en particulier la D-glucosamine et la D- galactosamine ;
- des acides aminés, en particulier la L- sérine ou tout extrait en contenant, en particulier des extraits d'algues ;
- de l'ascorbate de sodium (vitamine C) et de ses dérivés comme le phosphate, le palmitate. l'acétate ou le propionate ;
- de la vitamine A (appelé aussi rétinol), de ses esters et dérivés en particulier le palmitate, l'acétate et le propionate ;
- de l'acide madécassique, de l'acide asiatique et de leurs dérivés glycosylés ;
- des vitamines du groupe B, en particulier les vitamines B1, B6 et B12 et l'acide folique ;
- de la vitamine PP;
- de la forskoline, de ses dérivés et des extraits de coléus forskolii ;
- de l'acide salicylique et ses dérivés, en particulier ceux à chaînes lipophiles ;
- l'ecdystérone et ses dérivés, en particulier les acétates ;
- des rétinoïdes, en particulier l'acide rétinoïque, le rétinaldéhyde et le rétinylphosphate ;
- des oligo-éléments, tels que la silice et ses dérivés comme le silanol, le magnésium et ses sels, le manganèse et ses sels ;
- des extraits de Potentilla erecta;
- des extraits de fruits, en particulier de pomme et de citron, en particulier l'eau de pomme ou ses polyphénols ;
- des inhibiteurs des phosphodiestérases, en particulier les xanthines ;
- des extraits de Siegesbeckia orientalis ou de Daturoside ;
- des extraits de Centella asiatica enrichi en triterpènes ;
- des extraits de Bertholletia ;
- de l'acide chlorogénique, de l'acide caféique, de l'acide sinapique et de l'acide cafféoyl quinique ;
- des substances antiradicalaires, en particulier les oligomères procyanidoliques (OPC), les extraits de thé vert, de pépins de raisins, l'acide nordihydroguaiarétique (NDGA), les extraits de curcumine et les curcuminoides.

**14.** Procédé de traitement cosmétique destiné à stimuler la synthèse et la sécrétion des protéoglycannes (PG) et/ou des glycosaminoglycannes (GAG), **caractérisé en ce qu'**il comprend l'application d'une quantité cosmétiquement efficace d'un composé choisi dans le groupe constitué du D-xylose, de ses esters, notamment l'ester de l'acide D-galacturonique et les esters d'acides gras du D-xylose tels que définis dans la revendication 7 ou 8 et des oligosaccharides contenant du D-xylose, notamment des oligosaccharides tels que définis dans la revendication 9, pour obtenir la stimulation desdites synthèse et sécrétion, ledit composé étant contenu dans un excipient cosmétiquement acceptable.

**15.** Procédé selon la revendication 14, **caractérisé en ce que** ledit composé est présent dans la composition à une concentration comprise entre 0,01 et 5 % en poids, de préférence entre 0,01 et 0,5 % en poids.

**16.** Utilisation d'un ester du D-xylose, en particulier de l'ester de l'acide D-galacturonique, ou d'un des esters d'acide gras du D-xylose tel que défini dans la revendication 7 ou 8, ou d'un oligosaccharide contenant du D-xylose, en particulier d'un oligosaccharide tel que défini dans la revendication 9, comme agent cosmétique destiné à hydrater la couche basale de l'épiderme.

**17.** Utilisation d'un ester du D-xylose, en particulier de l'ester de l'acide D-galacturonique, ou d'un des esters d'acide gras du D-xylose tel que défini dans la revendication 7 ou 8, ou d'un oligosaccharide contenant du D-xylose, en particulier d'un oligosaccharide tel que défini dans la revendication 9, pour la fabrication d'une composition pharmaceutique, notamment dermatologique, destinée à traiter les insuffisances de l'hydratation de la couche basale de l'épiderme.

**Claims**

1. Cosmetic use of a compound selected from the group consisting of D-xylose, esters thereof and oligosaccharides containing D-xylose as a cosmetic agent stimulating the synthesis and/or secretion of proteoglycans (PG) and/or glycosaminoglycans (GAG) by the keratinocytes, especially the keratinocytes of the epidermis and the tissues with a keratinocyte covering compartment, such as mucous membranes, particularly the lips, and skin appendages, particularly the hair follicles, said agent being incorporated in a cosmetic composition comprising a cosmetically acceptable vehicle.

2. Use according to claim 1, **characterized in that** said cosmetic agent contributes to at least one of the following activities:

   - combating ageing of the epidermis;
   - combating drying of the skin associated with an insufficiency in the action of GAG, particularly hyaluronic acid;
   - improving the tonicity of the skin;
   - maintaining or restoring the suppleness and elasticity of the skin;
   - improving the mineralization of the epidermis, thereby making the skin healthier and improving its vitality;
   - facilitating intercellular exchanges;
   - improving the three-dimensional structure of the dermo-epidermal junction;
   - facilitating healing without scar formation, thus making it possible to repair epidermal microtraumatisms;
   - facilitating migration of the keratinocytes, allowing the formation of a corneal layer of good quality;
   - modulating the action of the growth factors and the cytokines produced by the skin cells.

3. Use of D-xylose and/or one of its esters for the manufacture of a pharmaceutical composition, especially dermatological composition, for treating insufficiencies in the synthesis or secretion of proteoglycans (PG) and/or glycosaminoglycans (GAG) by the keratinocytes, with a view to correcting the adverse effects of said insufficiencies and in particular to improving the functional state of the skin cells, especially the epidermal cells.

4. Use according to claim 3, **characterized in that** said pharmaceutical composition, especially dermatological composition, is intended to facilitate healing and repair epidermal microtraumatisms, or to treat skin ulcerations, especially ulcerations on the legs, or to reduce the striae distensae of pregnancy.

5. Use according to one of claims 1 to 4, **characterized in that** said compound is D-xylose.

6. Use according to one of claims 1 to 4, **characterized in that** said compound is a D-galacturonic acid ester.

7. Use according to one of claims 1 to 4, **characterized in that** said compound is an ester of a fatty acid comprising from 16 to 24 carbon atoms, particularly an ester of a natural fatty acid.

8. Use according to claim 7, **characterized in that** said fatty acid is selected from the group consisting of palmitic, stearic, oleic, linoleic, linolenic, arachidonic, erucic and lignoceric acids.

9. Use according to one of claims 1 to 4, **characterized in that** said compound is an oligosaccharide comprising at least one xylose and from 1 to 6 sugars and is selected from the group consisting of xylobiose, xylobiose hexaacetate, methyl-β-xylobioside, xylotriose, xylotetraose, xylopentaose and xylohexaose.

10. Use according to one of claims 1 to 9, **characterized in that** said compound is present in said composition at a concentration of between 0.001 and 5% by weight, preferably of between 0.01 and 0.5% by weight, based on the weight of said composition.

11. Use according to one of claims 1 to 10, **characterized in that** said composition is formulated for topical application.

12. Use according to one of claims 1 to 11, **characterized in that** said composition contains liposomes, said compound being either outside said liposomes or partially or totally incorporated inside said liposomes.

13. Use according to one of claims 1 to 12, **characterized in that** said composition also contains at least one other active principle selected from the group consisting of:

- amino sugars, particularly D-glucosamine and D-galactosamine;
- amino acids, particularly L-serine or any extract containing it, especially extracts of algae;
- sodium ascorbate (vitamin C) and derivatives thereof such as the phosphate, palmitate, acetate or propionate;
- vitamin A (also called retinol) and esters and derivatives thereof, particularly the palmitate, acetate and propionate;
- madecassic acid, asiatic acid and glycosylated derivatives thereof;
- B group vitamins, particularly vitamins B1, B6 and B12, and folic acid;
- vitamin PP;
- forskolin, derivatives thereof and extracts of Coleus forskolii;
- salicylic acid and derivatives thereof, particularly those with lipophilic chains;
- ecdysterone and derivatives thereof, particularly the acetates;
- retinoids, particularly retinoic acid, retinaldehyde and retinyl phosphate;
- micronutrients such as silica and derivatives thereof like silanol, magnesium and salts thereof and manganese and salts thereof;
- extracts of Potentilla erecta;
- extracts of fruits, particularly apple and lemon and especially apple juice or its polyphenols;
- phosphodiesterase inhibitors, particularly xanthines;
- extracts of Siegesbeckia orientalis or daturoside;
- extracts of Centella asiatica enriched in triterpenes;
- extracts of Bertholletia;
- chlorogenic acid, caffeic acid, sinapic acid and caffeoylquinic acid;
- free radical inhibitors, particularly procyanidolic oligomers (PCO), extracts of green tea and grape seeds, nordihydroguaiaretic acid (NDGA), curcumin extracts and curcuminoids.

14. Method of cosmetic treatment for stimulating the synthesis and secretion of proteoglycans (PG) and/or glycosaminoglycans (GAG), **characterized in that** it comprises the application of a cosmetically effective amount of a compound selected from the group consisting of D-xylose, esters thereof, especially the D-galacturonic acid ester and the fatty acid esters of D-xylose as defined in claim 7 or 8, and oligosaccharides containing D-xylose, especially oligosaccharides as defined in claim 9, for stimulating said synthesis and secretion, said compound being contained in a cosmetically acceptable excipient.

15. Method according to claim 14, **characterized in that** said compound is present in the composition at a concentration of between 0.01 and 5% by weight, preferably of between 0.01 and 0.5% by weight.

16. Use of a D-xylose ester, particularly the D-galacturonic acid ester or one of the fatty acid esters of D-xylose as defined in claim 7 or 8, or an oligosaccharide containing D-xylose, particularly an oligosaccharide as defined in claim 9, as a cosmetic agent for hydrating the basal layer of the epidermis.

17. Use of a D-xylose ester, particularly the D-galacturonic acid ester or one of the fatty acid esters of D-xylose as defined in claim 7 or 8, or an oligosaccharide containing D-xylose, particularly an oligosaccharide as defined in claim 9, for the manufacture of a pharmaceutical composition, especially dermatological composition, for treating insufficiencies in the hydration of the basal layer of the epidermis.

**Patentansprüche**

1. Kosmetische Verwendung einer Verbindung, die ausgewählt wird aus der Gruppe, die besteht aus D-Xylose, ihren Estern und Oligosacchariden, die D-Xylose enthalten, als kosmetisches Agens, das die Synthese und/oder Sekretion von Proteoglycanen (PG) und/oder Glycosaminoglycanen (GAG) durch die Keratinozyten, insbesoindere die Keratinozyten der Oberhaut und der Gewebe, die einen Abschnitt von Deck-Keratinozyten aufweisen wie die Schleimhäute, insbesondere die Lippen, und die Hautanhangsgebilde, insbesondere die Haarfollikel, stimuliert, wobei das genannte Agens einer kosmetischen Zusammensetzung einverleibt ist, die ein kosmetisch verträgliches Vehiculum enthält.

2. Verwendung nach Anspruch 1, **dadurch gekennzeichnet**, dass das genannte kosmetische Agens zu mindestens einer der folgenden Aktivitäten beiträgt:

- Bekämpfung der All:erung der Oberhaut;

- Bekämpfung der Austrocknung der Haut, die verbunden ist mit einer Insuffizienz der Wirkung der GAG, insbesondere der Hyaluronsäure,
- Verbesserung der SpannkraK der Haut,
- Aufrechterhaltung und Wiederherstellung der Weichheit und Elastizität der Haut,
- Verbesserung der Mineralisation der Oberhaut, um die Haut so gesünder zu machen und ihre Vitalität zu verbessern,
- Erleichterung der interzellulären Austauschvorgänge,
- Verbesserung der dreidimensionalen Struktur der Lederhaut-Oberhaut-Verbindungsstelle (-Grenze),
- Erleichterung der Vernarbung ohne Narbenbildung, um so die Reparatur von Oberhaut-Mikroverletzungen zu erlauben,
- Erleichterung der Wanderung der Keratinozyten, um so die Bildung einer Homschicht mit guter Qualität zu erlauben, und
- Modulation der Wirkung der Wachstumsfaktoren und der Cytokine, die von den Hautzellen gebildet werden.

3. Verwendung von D-Xylose und/oder eines ihrer Ester zur Herstellung einer pharmazeutischen, insbesondere dermatologischen Zusammensetzung für die Behandlung von Insuffizienzen der Synthese und der Sekretion von Proteoglycanen (PG) und/oder Glycosaminoglycanen (GAG) durch die Keratinozyten, um die negativen Effekte der genannten Insuffizienzen zu korrigieren, insbesondere den Funktionszustand der Zellen der Haut, insbesondere der Oberhaut, zu verbessern.

4. Verwendung nach Anspruch 3, **dadurch gekennzeichnet**, dass die genannte pharmazeutische, insbesondere dermatologische Zusammensetzung dafür bestimmt ist, die Vernarbung und die Reparatur von Oberhaut-Mikroverletzungen zu erleichtern oder Hautgeschwüre, insbesondere Beingeschwüre, zu behandeln oder Schwangerschafts-Dehnungsstreifen zu vermindern.

5. Verwendung nach einem der Ansprüche 1 bis 4, **dadurch gekennzeichnet**, dass die genannte Verbindung D-Xylose ist.

6. Verwendung nach einem der Ansprüche 1 bis 4, **dadurch gekennzeichnet**, dass die genannte Verbindung ein Ester der D-Galacturonsäure ist.

7. Verwendung nach einem der Ansprüche 1 bis 4, **dadurch gekennzeichnet**, dass die genannte Verbindung ein Fettsäureester mit 16 bis 24 Kohlenstoffatome, insbesondere ein Ester einer natürlichen Fettsäure, ist.

8. Verwendung nach Anspruch 7, **dadurch gekennzeichnet**, dass die genannte Fettsäure ausgewählt wird aus der Gruppe, die besteht aus Palmitinsäure, Stearinsäure, Ölsäure, Linolsäure, Linolensäure, Arachidonsäure, Erucasäure und Lignocerinsäure.

9. Verwendung nach einem der Ansprüche 1 bis 4, **dadurch gekennzeichnet**, dass die genannte Verbindung ein Oligosaccharid ist, das mindestens eine Xylose und 1 bis 6 Zucker enthält, das ausgewählt wird aus der Gruppe, die besteht aus Xylobiose, Xylobiosehexaacetat, Methyl-$\beta$-xylobiose, Xylotriose, Xylotetraose, Xylopentaose und Xylohexaose.

10. Verwendung nach einem der Ansprüche 1 bis 9, **dadurch gekennzeichnet**, dass die genannte Verbindung in der genannten Zusammensetzung in einer Konzentration zwischen 0,001 und 5 Gew.-%, vorzugsweise zwischen 0,01 und 0,5 Gew.-%, bezogen auf das Gewicht der genannten Zusammensetzung, vorliegt.

11. Verwendung nach einem der Ansprüche 1 bis 10, **dadurch gekennzeichnet**, dass die genannte Zusammensetzung für einen topischen Auftrag formuliert ist.

12. Verwendung nach einem der Ansprüche 1 bis 11, **dadurch gekennzeichnet**, dass die genannte Zusammensetzung Liposome enthält, wobei sich die genannte Verbindung entweder außerhalb der genannten Liposome oder teilweise oder vollständig im Innern der genannten Liposome befindet.

13. Verwendung nach einem der Ansprüche 1 bis 12, **dadurch gekennzeichnet**, dass die genannte Zusammensetzung außerdem mindestens einen weiteren Wirkstoff enthält, der ausgewählt wird aus der Gruppe, die besteht aus:

- Aminozuckern, insbesondere D-Glucosamin und D-Galactosamin;

- Aminosäuren, insbeaondere L-Serin, oder jedem diese enthaltenden Extrakt, insbesondere Algenextrakten;
- Natriumascorbat (Vitamin C) und seinen Derivaten wie dem Phosphat, Palmitat, Acetat oder Propionat;
- Vitamin A (auch als Retinol bezeichnet) und seinen Estern und Derivaten, insbesondere dem Palmitat, Acetat und Propionat;
- Madecassinsäure, Asiatsäure und ihren glycosylierten Derivaten;
- Vitaminen der Gruppe B, insbesondere den Vitaminen B1, B6 und B12 und Folsäure;
- Vitamin PP;
- Forskolin, seinen Derivaten und Extrakten von Coleus forskolii;
- Salicylsäure und ihren Derivaten, insbesondere denjenigen mit lipophilen Ketten;
- Ecdysteron und seinen Derivaten, insbesondere den Acetaten;
- Retinoiden, insbesondere Retinoesäure, Retinaldehyd und Retinylphosphat;
- Oligoelementen, wie Siliciumdioxid und seinen Derivaten wie Silanol, Magnesium und seinen Salzen, Mangan und seinen Salzen;
- Extrakten von Potentilla erecta;
- Extrakten von Früchten, insbesondere Äpfeln und Zitronen, insbesondere Apfelsaft, oder ihren Polyolen;
- Inhibitoren von Phosphodiesterasen, insbesondere Xanthinen;
- Extrakten von Siegesbeckia orientalis oder Daturoside;
- Extrakten von Centella asiatica, angereichert an Triterpenen;
- Extrakten von Bertholletia;
- Chlorogensäure, Kaffeesäure, Senfsäure und Kaffeeoylchininsäure;
- Antiradikal-Substanzen, insbesondere Procyanidol-Oligomeren (OPC), Extrakten von grünem Tee, von Traubenkemen, Nordihydroguajaretsäure (NDGA), Extrakten von Curcumin und Curcuminoiden.

14. Kosmetisches Behandlungsverfahren zur Stimulierung der Synthese und der Sekretion von Proteoglycanen (PG) und/oder Glycosaminoglycanen (GAG), **dadurch gekennzeichnet**, dass es umfasst das Auftragen einer kosmetisch wirksamen Menge einer Verbindung, ausgewählt aus der Gruppe, die besteht aus D-Xylose, ihren Estem, insbesondere dem Ester der D-Galacturonsäure, und Fettsäureestern von D-Xylose, wie sie in Anspruch 7 oder 8 definiert sind, und Oligosacchariden, die D-Xylose enthalten, insbesondere Oligosacchariden, wie sie im Anspruch 9 definiert sind, zur Erzielung der Stimulation der genannten Synthese und Sekretion, wobei die genannte Verbindung in einem kosmetisch verträglichen Exzipienten enthalten ist.

15. Verfahren nach Anspruch 14, **dadurch gekennzeichnet**, dass die genannte Verbindung in der Zusammensetzung in einer Konzentration zwischen 0,01 und 5 Gew.-%, vorzugsweise zwischen 0,01 und 0,5 Gew.-%, vorliegt.

16. Verwendung eines Esters von D-Xylose, insbesondere des Esters der D-Galacturonsäure oder eines der Fettsäureester von D-Xylose, wie sie in Anspruch 7 oder 8 definiert sind, oder eines Oligosaccharids, das D-Xylose enthält, insbesondere eines Oligosaccharids, wie es in Anspruch 9 definiert ist, als kosmetisches Agens für die Hydratation der Basalschicht der Oberhaut.

17. Verwendung eines Esters von D-Xylose, insbesondere des Esters der D-Galacturonsäure oder eines der Fettsäureester von D-Xylose, wie sie in Anspruch 7 oder 8 definiert sind, oder eines Oligosaccharids, das D-Xylose enthält, insbesondere eines Oligosaccharids, wie es in Anspruch 9 definiert ist, zur Herstellung einer pharmazeutischen, insbesondere dermatologischen Zusammensetzung für die Behandlung der Insuffizienzen der Hydratation der Basalschicht der Oberhaut.